# EUROPÄISCHE PATENTANMELDUNG

(11) **EP 1 731 532 A1**
(43) Veröffentlichungstag der Anmeldung: **13.12.2006**
(21) Anmeldenummer: 06010793.5
(22) Anmeldetag: 26.05.2006
(51) Int. Cl.: C08B 37/00

(54) **Depolymerisiertes Scleroglucan zur Regulierung und Verbesserung des Feuchtigkeitsgehaltes der Haut**

(30) Priorität: 07.06.2005 DE 102005026061
(71) Anmelder: Goldschmidt GmbH, 45127 Essen (DE)
(72) Erfinder: Farwick, Mike, Dr., 45138 Essen (DE); Maczkiewitz, Ursula, 45219 Essen (DE); Mecking, Maria, 46244 Bottrop (DE); Schick, Georg, Dr., Chester/Virginia 23831 (US); Wollenweber, Ute, 45130 Essen (DE)

(57) **Zusammenfassung**

Gegenstand der Erfindung ist die Verwendung von depolymerisiertem Scleroglucan allein oder in Kombination mit einem weiteren oder mehreren Wirkstoffen als Feuchthaltemittel sowie als entzündungshemmenden Wirkstoff zum Schutze und zur Wiederherstellung einer gesunden Hautbarriere auf dem Gebiet der kosmetischen oder dermatologischen Hautpflege.

## Beschreibung

Gegenstand der Erfindung ist die Verwendung von depolymerisiertem Scleroglucan allein oder in Kombination mit einem weiteren oder mehreren Wirkstoffen als Feuchthaltemittel sowie als entzündungshemmenden Wirkstoff zum Schutze und zur Wiederherstellung einer gesunden Hautbarriere auf dem Gebiet der kosmetischen oder dermatologischen Hautpflege.

Die Hornhaut (Stratum Corneum, SC), welche die äußerste Schicht der Haut darstellt, ist als wichtige Barriereschicht von besonderer Bedeutung für den Schutz vor Umwelteinflüssen. Zur Erhaltung ihrer Glätte, Elastizität und Geschmeidigkeit benötigt die Haut ein Optimum an Wasser. Diese Erkenntnisse wurden in grundlegenden Arbeiten u. a. von Jacobi sowie Schuleit und Szakall bestätigt (Jacobi, J. Appl. Physiol. 12 (3), 403-7, May 1958; Schneider W & Schuleit H, Arch. Klein. Exp. Dermatol. 193 (5), 434-59, Dec. 1951; Szakall A, Arch. Klein. Exp. Dermatol. 206, 374-9, 1957).

Der Mensch gibt täglich mehrere Deziliter bis zu mehreren Litern Wasser über die Haut an die Umwelt ab. Das in der Haut befindliche Wasser stammt aus verschiedenen Quellen und liegt nach neueren Erkenntnissen sowohl als Dampf als auch in flüssiger Form sowie adsorbiert an Proteinen vor. Es ist nicht bekannt, wie viel Wasser die Epidermis enthält, man kann aber davon ausgehen, dass in einigen Schichten des Stratum Corneums ein Wasseranteil von bis zu 30 % vorliegt.

Als sicher angenommen werden kann, dass Wasser befähigt ist, durch verschiedene Hautschichten zu wandern. Für die Diffusion des Wassers durch die Hautschichten gibt es dabei verschiedene Modelle, von denen bis jetzt noch keines schlüssig bewiesen werden konnte:
Analog zu hydrophoben Substanzen, welche durch Lipidporen in die Hornschicht penetrieren können, soll das Wasser durch spezifische Wasserporen, sog. "aqueous pores", transportiert werden. Diese Poren sollen einen Durchmesser von 15-25 Ä besitzen.

Ein anderer Ansatz postuliert, dass das Stratum Corneum von wassergefüllten Kanälen durchzogen sein soll. Durch Beugungsexperimente mit Röntgenstrahlen konnte gezeigt werden, dass in einem Lipid-Doppelschichtsystem Lücken bestehen, die groß genug sind, um kondensiertes Wasser dort ansammeln zu können.

Zur Feuchtigkeitsregulation der Haut ist also unzweifelhaft neben einer intakten Permeabilitätsbarriere das Vorhandensein von wasserbindenden Substanzen maßgeblich erforderlich, die in den epidermalen Hornschichten gebildet werden. Diese in der Epidermis enthaltenen natürlichen Feuchthaltestoffe NMF (natural moisturizing factors) binden Feuchtigkeit in der Haut. Sie stellen ein Gemisch verschiedener Verbindungen dar und bestehen aus 40 % Aminosäuren, 12 % Pyrrolidoncarbonsäure, 7 % Harnstoff sowie 41 % anorganischen und organischen Salzen (hauptsächlich Lactate).

Drastische Umweltbedingungen, wie z. B. niedrige Temperaturen oder zu geringe Feuchtigkeit im Winter, tragen in erheblichem Maße mit dazu bei, dass die Haut rau und trocken wird. Die in der Epidermis enthaltenen Feuchthaltestoffe werden zudem leicht durch häufiges Waschen oder Baden herausgelöst. So kann mehr Wasser aus tiefer gelegenen Hautschichten entweichen und der so genannte transepidermale Wasserverlust (TEWL) nimmt zu, was eine Austrocknung der Haut bewirkt. Es wird angenommen, dass der Verlust der natürlichen Feuchthaltestoffe mit einer Verringerung des Wassergehalts und einer reduzierten Weichheit der Keratinschicht korreliert.

Sensorisch manifestiert sich dies durch Symptome wie z. B. eine vermehrt raue, schuppige, glanzlose und stumpf wirkende Hautoberfläche. Ein Flexibilitätsverlust und eine Beeinträchtigung der Barrierefunktion der Haut, die von der Wasserbindungskapazität des Stratum Corneums abhängt, sind die Folge. Dadurch wird der Wassergehalt der Hornschicht weiter reduziert.

Eine sorgfältige Pflege zur Verhinderung einer andauernd trokkenen Haut ist nicht nur ein ästhetisches Bedürfnis, sondern auch ein probates Mittel, um chronischen Hautkrankheiten effektiv vorzubeugen. Hierbei kann die Feuchtigkeitsregulierung der Haut durch topische Applikation entsprechender Formulierungen wirkungsvoll unterstützt werden.

Eine Vielzahl von in vivo-Methoden zur Ermittlung des Feuchtigkeitsgehaltes der Haut sind bekannt. Dabei werden physikalische Parameter wie die Leitfähigkeit und die dielektrischen Eigenschaften (Kapazität) der Hornschicht bestimmt, die direkt mit der Hautfeuchtigkeit korrelieren. Zur Bestimmung der Hydratation des Stratum Corneums stehen verschiedene Messgeräte zur Verfügung, wie z. B. die Corneometer-Typen CM 820 und CM 825 (Courage + Khazaka) sowie das "dermal phase meter" Skicon 200 (Nova). Diese nicht-invasiven und einfachen Methoden erlauben es, eine Veränderung der Hautfeuchtigkeit quantitativ zu messen. Darüber hinaus kann die Viscoelastizität der Haut über das Dermal Torque Meter (DiaStron) oder auch über das Cutometer (Courage + Khazaka) ermittelt werden.

Um einem trockenen Hautzustand entgegenzuwirken und den Wasserhaushalt der Haut wiederherzustellen, gibt es eine Reihe von kosmetischen Zubereitungen mit hydroregulativer Wirkung. Diese Präparate sind in Form von Emulsionen ideale Zubereitungen, um der Haut Fett und Feuchtigkeit zuzuführen und enthalten in der Regel eine Reihe von Wirkstoffen, die beim Auftragen eine schützende Funktion entfalten, dadurch den Zustand der Hautoberfläche verbessern und den funktionellen Zustand der Haut verändern, indem sie z. B. regulierend auf die Hautfeuchte einwirken und durch das Eindringen unter die Hautoberfläche pflegende Eigenschaften zur Wirkung kommen.

Es existieren verschiedene Mechanismen zur positiven Beeinflussung des epidermalen Wassergehaltes durch kosmetische Inhaltsstoffe und Formulierungen:
Das Verdampfen von Wasser aus den oberen Hautschichten kann durch einen okklusiven Lipid- oder Polymerfilm unterbunden werden. Dadurch wird von den unteren Hautschichten Wasser an die oberen abgegeben sowie die Schweißbildung vermindert, wodurch die Hautfeuchtigkeit der oberen Schichten des SC stark ansteigt. Unter solchen okklusiven Bedingungen kommt es aber typischerweise zu einem Wasserstau in der Haut und einer vermehrten endogenen Quellung der Hornschicht, wodurch die Regenerationsfähigkeit der Haut verlangsamt wird.

Formulierungstechnisch ist es möglich, kosmetische Produkte herzustellen, die mehr Wasser enthalten als das Stratum Corneum und somit bei Penetration der intakten Formulierung Wasser an das SC abgeben. Spezielle Lipide sind ebenfalls in der Lage den transepidermalen Wasserverlust zu reduzieren und können daher auch als eine Art Moisturizer angesehen werden.

Ein weiterer gebräuchlicher Ansatz ist der Zusatz von Feuchthaltemitteln als aktivierende Inhaltsstoffe zu kosmetischen Emulsionen, welche die Versorgung der Keratinschicht mit einer ausreichenden Menge Feuchtigkeit über definierte Zeitabschnitte sicherstellen sollen. Feuchthaltemittel werden auch als Moisturizer oder Humectants bezeichnet und sollen einerseits Wasser in der Epidermis zurückhalten, andererseits durch Stabilisierung der Barrierefunktion in der oberen Hornschicht den TEWL vermindern.

Eine Vielzahl solcher Substanzen ist beschrieben und wird bereits verwendet. Diese besitzen in der Regel die Fähigkeit, Wasser mehr oder weniger stark zu binden und die ausgewaschenen natürlichen Stoffe ganz oder teilweise zu ersetzen. Prinzipiell gehören dazu hygroskopische Substanzen wie vor allen Dingen mehrwertige Alkohole, ethoxylierte Polyole, Zucker sowie Polysaccharide, wie z. B. das hauteigene Feuchthaltemittel Hyaluronsäure und seine Salze, die eine wichtige Rolle bei der Feuchtigkeitsregulation inne hat, da sie Wasser im Stratum Corneum binden kann. Dies hat schließlich eine Verbesserung der Hautelastizität zur Folge.

Scleroglucan (INCI: Sclerotium Gum, chemisch: β-1,3-Glucan) ist ein Polysaccharid mikrobiologischen Ursprungs. Jede dritte Glukoseeinheit ist über eine β-1,6-Verbindung mit einer weiteren Glucose verknüpft (allgemeine Formel). In der nativen Form bildet Scleroglucan im neutralen Milieu die für β-Glucan typische Dreifachhelix mit einem Molekulargewicht von 3-5 x 10⁶ aus. Die maximale Löslichkeit in Wasser bei neutralem bis schwach saurem pH liegt bei ca. 1,5 - 2 %.

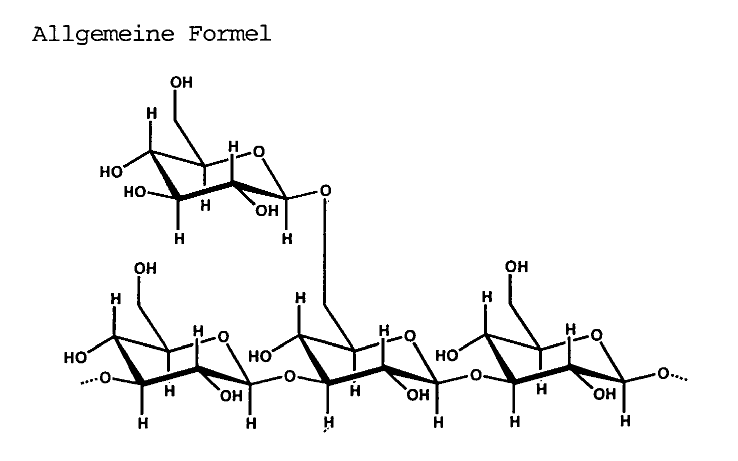

Die stark verdickenden Eigenschaften von Scleroglucan sind weithin bekannt und in verschiedenen Patenten beschrieben (EP 0 979 642, WO 00/61098, JP 612 675/03).

Aufgrund der antiviralen sowie antibakteriellen Wirkung von β-Glucanen wurden außerdem Anwendungen im medizinischen sowie Ernährungsbereich dieser Biopolymerklasse beschrieben, z. B. WO 98/04082, GB 2 050 825. Es ist allgemein bekannt, dass β-Glucane immunstimulierende Wirkungen zeigen, weshalb diese Stoffklasse attraktive Wirkstoffe für Anti-Aging und Sun Care-Anwendungen sind (WO 98/04082).
Neben der verdickenden Eigenschaft wurde im Patent US 6 162 449 die Verbesserung der Hauthydratation nach topischer Applikation einer Scleroglucan enthaltenden kosmetischen Formulierung vorgestellt. Das beschriebene Scleroglucan besitzt ein mittleres Molekulargewicht von 1-12 x 10⁶. Neben Hautfeuchtigkeit werden hier auch die entzündungshemmende Wirkung sowie das angenehme Hautgefühl der Endformulierung ausgelobt. Die Produktform ist eine 1 %ige Lösung, die bis zu 10 % der Formulierung zugegeben werden kann.

Es wurde nun überraschenderweise gefunden, dass depolymerisiertes Scleroglucan verglichen mit dem hochmolekularen Derivat sehr viel größere Effektivität im Bereich der Hauthydratation aufweist.

Darüber hinaus kann das depolymerisierte Scleroglucan auf Grund seines geringeren Molekulargewichtes und der damit einhergehenden niedrigeren Viskosität letztendlich in höheren Konzentrationen in die Endformulierung eingebracht werden.

Es wurde weiterhin überraschenderweise gefunden, dass depolymerisiertes Scleroglucan entzündungshemmende Aktivität besitzt und somit für Anwendungen in anti-inflammatorischen und After Sun-Hautpflegepräparationen geeignet ist.

Dementsprechend stellt die vorliegende Erfindung ein β-1,3-Scleroglucan des mittleren Molekulargewichtes von 8 x 10² bis 1 x 10⁶ , vorzugsweise 1 x 10⁵ bis 7 x 10⁵, vor, das mit einem geeigneten Träger in einer Endkonzentration von 0,05 bis 6,0, vorzugsweise 0,2 bis 2,0 Gew.-%, in die Endformulierung inkorporiert werden kann.

Die kosmetische Zusammensetzung entsprechend der vorliegenden Erfindung kann eine Hautpflegeformulierung wie z. B. eine Emulsion oder eine Creme sein, in denen das Scleroglucan eine oder mehrere Wirksamkeiten aufweist, wie z. B. eine Verbesserung des Feuchtigkeitsgehaltes sowie filmbildende und damit schützende Eigenschaften auf der Haut. Der filmbildende Effekt spendet der Haut außerdem ein weiches und seidiges Gefühl.

Die Hautpflegeformulierung kann als wässrige Lotion, Wasser-in-Öl- oder Öl-in-Wasseremulsion, Öl- oder Öl-Alkohollotion, vesikulare Dispersion anionischer oder nichtionischer amphiphiler Lipide, wässrige, Wasser-Alkohol, Alkohol- oder Öl-Alkoholgel, fester Stick oder als Aerosol formuliert werden.

Wenn sie als Wasser-in-Öl oder Öl-in-Wasser-Emulsion formuliert wird, enthält der kosmetisch annehmbare Träger vorzugsweise 5 bis 50 % einer Ölphase und 47 bis 94,95 % Wasser, bezogen auf dem Gesamtanteil der Formulierung.

Die Ölphase kann jedes kosmetische Öl oder eine Mischung davon enthalten. Beispiele solcher Öle beinhalten aliphatische Kohlenwasserstoffe wie flüssiges Paraffin, Squalan, Vaseline und Ceresin, Pflanzenöle wie Olivenöl, Mandelöl, Sesamöl, Avocadoöl, Rizinusöl, Kakaobutter und Palmöl, Tieröle wie Haifischlebertran, Dorschleberöl, Walöl, Rindertalg und Butterfett, Wachse wie Bienenwachs, Karnaubapalmwachs, Spermazet und Lanolin, Fettsäuren wie Laurinsäure, Myristinsäure, Palmitin-säure, Stearinsäure, Ölsäure und Behensäure; aliphatische Alkohole wie Lauryl-, Stearyl-, Cetyl- und Oleylalkohol und aliphatische Ester wie Isopropyl-, Isocetyl- oder Octadecylmyristat, Butylstearat, Hexyllaurat, Diisopropylester der Adipinsäure oder Diisopropylsebacat. Bevorzugte Mono- oder Polyole für die Verwendung in Öl-Alkohol-Lotionen oder Öl-Alkohol- oder Alkoholgel, beinhalten Ethanol, Isopropanol, Propylenglykol, Hexylenglykol, Glycerin und Sorbit.

Kosmetische Formulierungen entsprechend der vorliegenden Erfindung verfügen über einen feuchtigkeitsspendenden und hautberuhigenden Effekt.

Neben den auf dem kosmetischen und dermatologischen Gebiet üblichen Formulierungsbestandteilen können auch biogene Wirkstoffe oder Wirkstoffkombinationen mitverwendet werden. Unter biogenen Wirkstoffen sind beispielsweise Tocopherol, Toco-pherolacetat, Tocopherolpalmitat, Ascorbinsäure, Desoxyri-bonucleinsäure, Retinol, Bisabolol, Allantoin, Phytantriol, Panthenol, α-Hydroxy-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe zu verstehen.

Neben Hautpflegeprodukten der oben beschriebenen Zusammensetzung kann es sich hierbei auch um Haarpflegeformulierungen wie Shampoos und/oder Konditionierer handeln, in die das Scleroglucan inkorporiert wird und eine mildernde Wirkung auf durch chemische Behandlung gereizte Kopfhaut ausübt.

Hochmolekulares Scleroglucan mit einem Molekulargewicht von 1 x 10⁶ bis 12 x 10⁶ wird durch Fermentation von Sclerotium rolfsii ATCC 15205 produziert. Hierzu wird der Pilz Sclerotium rolfsii ATCC 15205 im Kulturmedium unter mikro-aeroben Bedingungen kultiviert. Das Medium enthält hierbei eine Kohlenstoffquelle, vorzugsweise Glucose, eine Stickstoffquelle, eine Phosphorquelle, Kaliumchlorid, Magnesiumsulfat-Hepta-hydrat, Eisen(II)-sulfat-Heptahydrat, sowie Hefeextrakt.

Das hochmolekulare Scleroglucan wird schließlich isoliert, indem der Kultivierungsprozess durch Agitation bei einer Temperatur von 15 - 40 °C beendet und die Zellmasse von der Flüssigkeit getrennt wird. Das hochmolekulare Scleroglucan kann zur Depolymerisierung als Lösung durch eine Kapillare gedrückt, so dass die Polysaccharidketten auf Grund von Scherkräften zerkleinert werden. Geeignete Lösungsmittel für diesen Prozess sind Wasser, Aceton oder Benzol im Gemisch mit organischen Solventien wie Methanol, Ethanol, iso- oder n-Propanol und Tetrahydrofuran. Der Grad der Depolymerisierung ist von verschiedenen physikalischen Parametern wie Druck sowie Durchmesser und Länge der Kapillare abhängig. Ein definierter Depolymerisationsgrad wird durch Wiederholung der Prozedur erzielt.

Alternativ kann eine Depolymerisation des Scleroglucans z. B. auch durch Inkubation des hochmolekularen Derivates zusammen mit einer wässrigen Lösung von Natriumhydroxid und Wasserstoffperoxid erlangt werden.

Das depolymerisierte Scleroglucan hat nach der beschriebenen Behandlung die dreidimensionale Struktur einer Tripelhelix, bestehend aus β-1,3-gebundener Glucopyranose als Hauptkette und β-1,6-gebundener Glucopyranose als Seitenketten mit einem mittleren Molekulargewicht von 8 x 10² bis 1 x 10⁶ , bevorzugt 1 x 10⁵ bis 7 x 10⁵, besonders bevorzugt 2 x 10⁵ bis 6 x 10⁵. Die dreideimensionale Struktur des depolymerisierten β-1,3-Scleroglucans kann experimentell z.B. mittels Lichtbeugungstechnik oder Gelfiltration bestimmt werden.

### Formulierungsbeispiele

Die Herstellung der erfindungsgemäßen Formulierungen erfolgt in der üblichen Weise, wobei das depolymerisierte Scleroglucan bevorzugt in der wässrigen Phase der Formulierung gelöst wird. Typische Rahmenrezepturen für Hautbehandlungsmittel gehören zum bekannten Stand der Technik und sind beispielsweise in den Broschüren der Hersteller der jeweiligen Grund- und Wirkstoffe enthalten. Scleroglucan kann dabei generell in einer Konzentration von 0,05 bis 6,0 Gew.-% enthalten sein.

Eine typische Emulsion (W/O oder O/W) enthält beispielsweise
0,05 bis 6,0 Gew.-% depolymerisiertes Scleroglucan,
0 bis 10 Gew.-% eines oder mehrerer Emulgatoren,
0 bis 10 Gew.-% eines oder mehrerer Konsistenzgeber,
0 bis 30 Gew.-% eines oder mehrerer kosmetischer Öle oder Emollients
sowie übliche Hilfs- und Zusatzstoffe in üblichen Konzentrationen.

### Effektivitätsnachweise zur näheren Erörterung der Erfindung

Um etwaige Effekte der in kosmetischen Rezepturen enthaltenen Ingredienzien zu vermeiden, wurde für die Durchführung der Wirksamkeitsnachweise eine Standardformulierung hergestellt, die in Tab. 1 zusammengefasst ist. Um außerdem den Einfluss des Polymerisationsgrades des in der vorliegenden Erfindung vorgestellten Scleroglucans zu untersuchen, wurden Produkte verschiedener Kettenlänge verwendet (s. hierzu Tab. 2).

**Tab. 1: O/W Creme**

| | |
|---|---|
| Ceteareth-25 | 2.0% |
| Glyceryl Stearate | 4.0% |
| Stearyl Alcohol | 2.0% |
| Ethylhexyl Stearate | 8.5% |
| Caprylic/Capric Triglyceride | 8.5% |
| Konservierungsmittel | 0.1% |
| Wasser | ad 100.0% |
| Scleroglucan | *x* % |

**Tab. 2: Depolymerisiertes Scleroglucan verschiedener Molekulargewichte**

| | MW* |
|---|---|
| Natives Scleroglucan | 2 x 10⁶ |
| Depolymerisiertes Produkt 1 | 520 000 |
| Depolymerisiertes Produkt 2 | 500 000 |
| Depolymerisiertes Produkt 3 | 400 000 |
| Depolymerisiertes Produkt 4 | 375 000 |

| | |
|---|---|
| *MW = Mol Weight | |

Die folgenden Beispiele sollen den Gegenstand der Erfindung näher erläutern:

### Beispiel 1: Bestimmung des Wasserrückhaltevermögens mittels eines artifiziellen Trägermaterials

Das Wasserrückhaltevermögen wird mit Hilfe eines Trägermaterials (Vitro Skin™ Substrate, IMS Incorp., Milford, USA) bestimmt, auf das nach Evaluation des Nettogewichtes die zu testende kosmetische Formulierung aufgetragen und abermals gewogen wird.

Die Applikation der Formulierung (Tab. 1, enthaltend 2 % der Produkte 1 - 4 bzw. das native Scleroglucan) erfolgt durch Betupfen des Substrates mit einem Spatel und anschließendem Verstreichen. Im Anschluss werden die Proben 4 Stunden bei 22 °C und 72 % relativer Feuchtigkeit inkubiert und schließlich erneut gewogen, wobei die Ein- und Auswaage in der Klimakammer unter definierten Bedingungen (22 °C, 55 % relative Feuchtigkeit) erfolgt. Der relative Gewichts(= Wasser)verlust kann rechnerisch ermittelt werden.

Die in Abb. 1 gezeigten Mittelwerte setzen sich aus mindestens 15 Einzelbestimmungen zusammen. 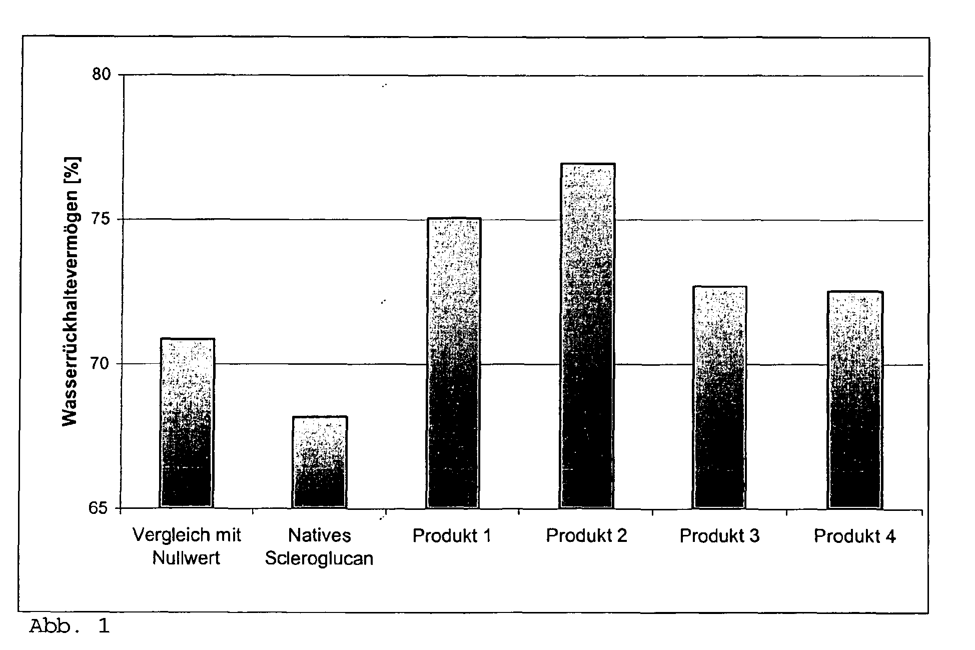 Abb. 1 verdeutlicht das effektive Wasserrückhaltevermögen von depolymerisiertem Scleroglucan, insbesondere von Produkt 2, verglichen mit einer Kontrollformulierung ("Vergleich mit Nullwert") sowie dem polymeren Ausgangsmaterial.

### Beispiel 2: Bestimmung der Hautfeuchtigkeit in vivo mittels Corneometrie

Die Corneometrie stellt eine nicht-invasive Methode dar, die auf Grund Veränderungen physikalischer Parameter im Stratum Corneum Rückschlüsse auf die Hautfeuchtigkeit gestattet. Zur praktischen Durchführung werden 12 bis 15 Probanden rekrutiert, deren Hautfeuchtigkeit vor und nach einmaliger Applikation der Testformulierungen mittels eines Corneometers HM99 (Courage & Khazaka, Köln, Deutschland) nach Angaben des Herstellers gemessen wird.

Nach einer Klimatisierung der Probanden über 15 Minuten unter definierten Bedingungen von 22 °C und 55 % relativer Feuchtigkeit wird die Hauthydratation corneometrisch bestimmt und im Anschluss daran 50 mg der Testformulierung (Tab. 1, enthaltend 1 % Produkt 1 - 4 sowie das native Ausgangsmaterial) auf einer Hautfläche mit einem jeweiligen Durchmesser von 2,5 cm aufgetragen und nach zwei Minuten etwaige Reste der Formulierung entfernt.

Nach einer zweistündigen Einwirkzeit wird erneut die Hauthydratation nach einer Klimatisierung von 15 Minuten (22 °C, 55 % rel. Feuchtigkeit) gemessen. Ein Anstieg der relativen Corneometereinheiten CU korreliert direkt mit einer verbesserten Hauthydratation (Abb. 2).

Die Abbildung verdeutlicht, dass depolymerisiertes Scleroglucan, speziell Produkt 4, verglichen mit dem nativen Ausgangsmaterial eine sehr viel größere Effektivität im Bereich der Hauthydratation aufweist.

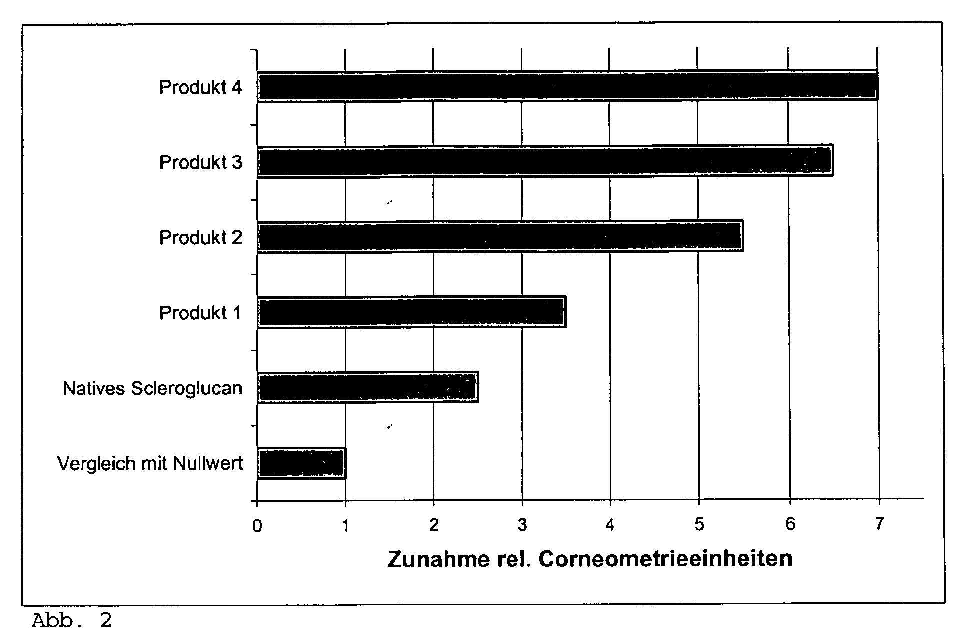

### Beispiel 3: Bestimmung der entzündungshemmenden Effektivität mittels Kurativstudie

In einer beim Institut Dr. Schrader (Holzminden, D) durchgeführten Kurativstudie wird die anti-inflammatorische Wirksamkeit des depolymerisierten Scleroglucans Produkt 4 verglichen mit dem polymeren Ausgangsmaterial demonstriert. Hierzu werden 20 Probanden rekrutiert, die über einen Zeitraum von 3 Tagen vor Beginn sowie während des Tests die Versuchsformulierungen (s. Tab. 1) enthaltend 0,5 % Wirkstoff auf die später bestrahlten Hautareale applizieren. Als Testareal dient der Rücken. Zunächst wird die MEDu (Minimale erythemale Dosis der unbehandelten Haut) der Probanden ermittelt und am darauf folgenden Tag die Ausgangssituation mittels eines Chromameters sowie eines Spektralphotometers dokumentiert.

Anschliessend (= Tag 0) erfolgt die Bestrahlung der Haut mit UV-Licht unter Anwendung des Sonnensimulators SU 5000 nach Schrader entsprechend des COLIPA-Bestrahlungsspektrums. Die Bestrahlungsdosis ist auf das 1,75fache der MEDu festgesetzt und bewirkt eine leichte Erythemreaktion der Haut. Nach der Bestrahlung werden die Probanden angewiesen, zweimal täglich die Testformulierungen auf die dafür vorgesehenen Areale zu applizieren.

Parallel zur täglichen Produktanwendung werden jeweils remissionsspektroskopische sowie Farbmessungen im Institut durchgeführt. So kann die Hautrötung vor sowie 24 und 48 Stunden nach der Bestrahlung in Form von Remission-Absorptionsintegralen quantitativ ermittelt werden.

Abb. 3 zeigt die Hautrötung in % an Tag 1 (= 24 Stunden nach Bestrahlung), an dem das Erythem sein Maximum erreicht, sowie an Tag 2. Hiernach weist das depolymerisierte Scleroglucan Produkt 4 verglichen mit dem nativen Ausgangsmaterial eine entzündungshemmende Effektivität auf, da die Hautrötung erkennbar schneller abklingt.

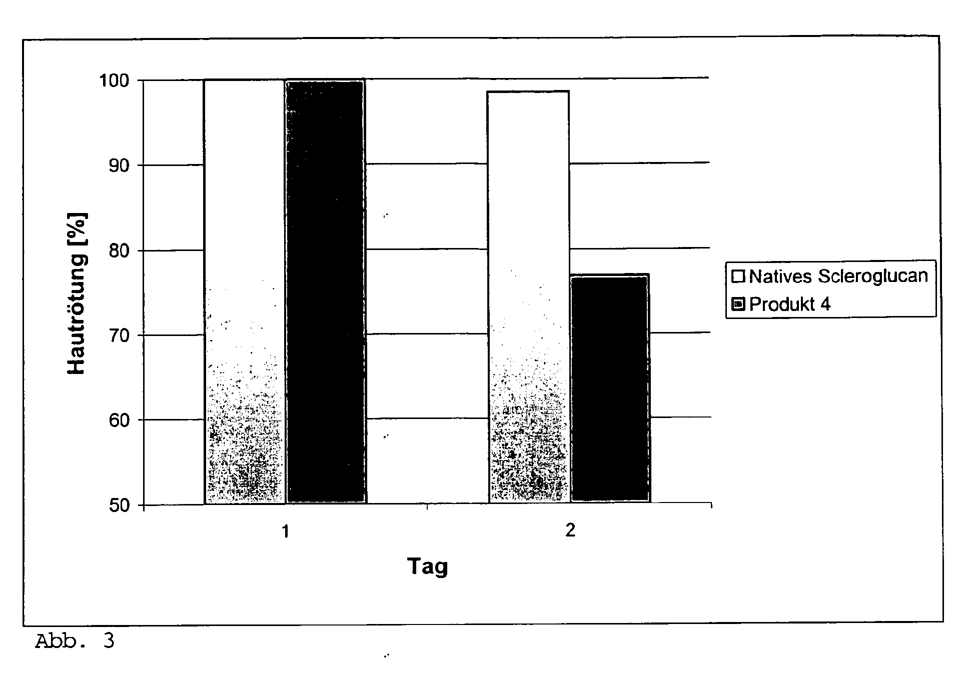

### Beispiel 4: Bestimmung der entzündungshemmenden Effektivität am künstlichen Hautmodell

In einer *in vitro-* Studie am künstlichen Hautmodell SkinEthic™ (SkinEthic Laboratories, Nizza, F) wird die anti-inflammatorische Wirksamkeit des depolymerisierten Scleroglucans Produkt 4 untersucht.

Hierzu werden zu Beginn der Versuchsreihe jeweils 30 µL entweder 0,9 %ige Natriumchlorid-Lösung ("Vergleich mit Nullwert") bzw. Produkt 4 in einer Konzentration von 0,1 mg/ mL bzw. 1 mg/ mL 24 Stunden lang auf die künstliche Haut appliziert.

Um eine Entzündungsreaktion am Hautmodell hervor zu rufen, werden im Anschluss daran 30 µL einer 0,25 %igen wässrigen Natrium-Dodecylsulfat-Lösung aufgetragen und erst nach 40 Minuten mit Medium zur Kultivierung der künstlichen Haut abgewaschen. Natrium-Dodecylsulfat (Sodium Dodecylsulfate; SDS) ist als stark hautreizendes Detergenz bekannt. Im Anschluss an die Schädigung werden wiederum jeweils 30 µL entweder 0,9 %ige Natriumchlorid-Lösung ("Vergleich mit Nullwert") bzw. Produkt 4 in einer Konzentration von 0,1 mg/ mL bzw. 1 mg/ mL auf die künstliche Haut appliziert.

Als Parameter der Zellschädigung wird das Enzym Lactatdehydrogenase (LDH) bestimmt, das bei geschädigten Zellen aus dem Cytosol frei gesetzt wird und so fotometrisch im Zellüberstand nachgewiesen werden kann. Die Zellschädigung wird direkt im Anschluss an die SDS-Applikation sowie 24 Stunden danach bestimmt.

Abb. 4 zeigt die LDH-Freisetzung 24 Stunden nach Applikation auf die Hautmodelle. Hiernach weist das depolymerisierte Scleroglucan Produkt 4 bereits in einer Konzentration von 0,1 mg/ mL eine deutlich entzündungshemmende Effektivität auf, da die LDH-Ausschüttung auf ungefähr die Hälfte reduziert werden kann.

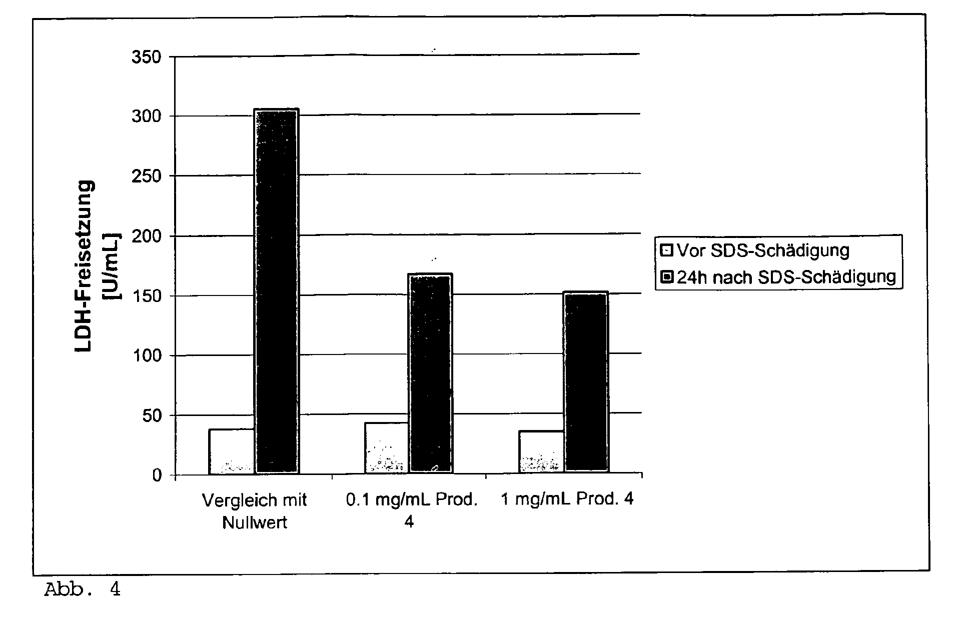

## Patentansprüche

1. β-1,3-Scleroglucan der allgemeinen Formel 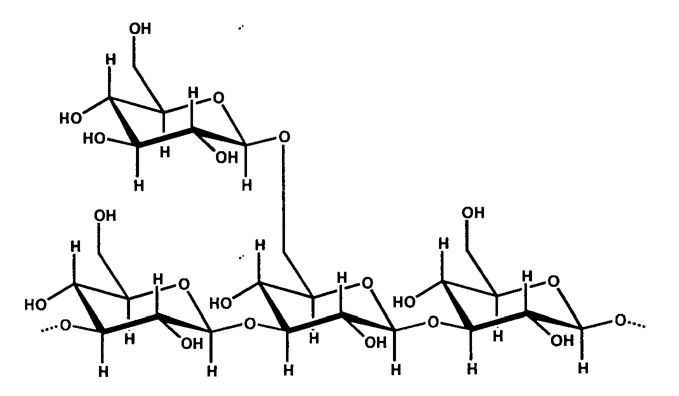 und einem mittleren Molekulargewicht von 8 x 10² bis 1 x 10⁶, vorzugsweise 1 x 10⁵ bis 7 x 10⁵.

2. Kosmetische und/oder dermatologische Formulierungen zur topischen Behandlung der Haut und Hautanhangsgebilde, enthaltend als Wirkstoff 0,05 bis 6,0 Gew.-% eines β-1,3-Scleroglucan der allgemeinen Formel 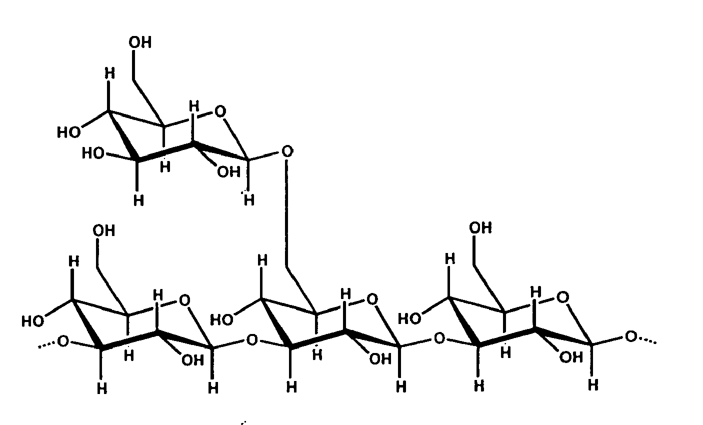 und einem mittleren Molekulargewichtsbereich von 8 x 10² bis 1 x 10⁶.

3. Kosmetische und/oder dermatologische Formulierungen gemäß Anspruch 2, enthaltend 0 bis 10 Gew.-% eines oder mehrerer Emulgatoren, 0 bis 10 Gew.-% eines oder mehrerer Konsistenzgeber, 0 bis 30 Gew.-% eines oder mehrerer Tenside, 0 bis 30 Gew.-% eines oder mehrerer kosmetischer Öle oder Emollients sowie gängige Hilfs- und Zusatzstoffe in üblichen Konzentrationen.

4. Kosmetische und/oder dermatologische Formulierungen gemäß Anspruch 2 und/oder 3 zur Regulierung und Verbesserung des Feuchtigkeitsgehalts der Haut.

5. Kosmetische und/oder dermatologische Formulierungen gemäß Anspruch 2 und/oder 3 zur anti-inflammatorischen Behandlung der Haut.

6. Kosmetische und/oder dermatologische Formulierungen gemäß Anspruch 2 und/oder 3 zur Herstellung von After-Sun-Hautpflegepräparationen.

7. Kosmetische und/oder dermatologische Formulierungen gemäß Anspruch 2 und/oder 3 zur Herstellung von Haarbehand-lungs- und Haarnachbehandlungsmitteln.

8. Kosmetische und/oder dermatologische Formulierungen gemäß mindestens einem der Ansprüche 2 bis 7, enthaltend mindestens eine Verbindung ausgesucht aus der Gruppe Tocopherol und Derivate, Ascorbinsäure und Derivate, Desoxyribonucleinsäure, Retinol und Derivate, Alpha-Liponsäure, Niacinamid, Ubichinon, Bisabolol, Allan-toin, Phytantriol, Panthenol, α-Hydroxy-Säuren, Aminosäuren, Hyaluronsäure, Polyglutaminsäure, Creatin und Creatinderivate, Guanidin und Guanidinderivate, Ceramide, Sphingolipide, Phytosphingosin und Phytosphingosinderivate, Sphingosin und' Sphingosinderivate, Sphinganin und Sphinganinderivate, Pseudo-Ceramiden, essentielle Ölen, Peptiden, Proteinen, Proteinhydrolysaten, Pflanzenextrakten und Vitaminkomplexen.
